# EUROPEAN PATENT SPECIFICATION

(11) **EP 3 336 199 B1**
(45) Date of publication and mention of the grant of the patent: **29.09.2021**
(21) Application number: 16836624.3
(22) Date of filing: 12.08.2016
(51) Int. Cl.: C12Q 1/6869, C12M 1/38, C12M 1/36, C12M 1/34, B01L 3/00

(54) **SINGLE MOLECULE SEQUENCING METHOD, DEVICE, SYSTEM, AND APPLICATION**
EINZELMOLEKÜLSEQUENZIERUNGSVERFAHREN, -VORRICHTUNG, -SYSTEM UND ANWENDUNG
PROCÉDÉ DE SÉQUENÇAGE À MOLÉCULE UNIQUE, DISPOSITIF, SYSTÈME, ET APPLICATION

(30) Priority: 14.08.2015 CN 201510501300
(43) Date of publication of application: 20.06.2018
(73) Proprietor: GeneMind Biosciences Company Limited, Luohu District, Shenzhen City, 518023 (CN)
(72) Inventor: GE, Liangjin, Shenzhen City Guangdong (CN); GAO, Yan, Shenzhen City Guangdong (CN); DENG, Liwei, Shenzhen City Guangdong (CN); WU, Zengding, Shenzhen City Guangdong (CN); CAI, Jinsen, Shenzhen City Guangdong (CN); JI, Daorui, Shenzhen City Guangdong (CN); ZENG, Lidong, Shenzhen City Guangdong (CN); LI, Gailing, Shenzhen City Guangdong (CN)
(74) Representative: Schiweck Weinzierl Koch Patentanwälte Partnerschaft mbB
(86) International application number: PCT/CN2016/095053
(87) International publication number: WO 2017/028760

(56) References cited:
- WO-A1-00/70073
- WO-A2-2005/080605
- WO-A2-2009/097626
- CN-A- 1 932 033
- CN-A- 105 112 516
- US-A1- 2009 082 212

### SEQUENCE LISTING

<110> Direct Genomics Co.,Ltd
<120> SINGLE MOLECULE SEQUENCING METHOD, APPARATUS, SYSTEM AND APPLICATION THEREOF
<130> DGE16296PCTEP
<150> CN201510501300.2
   <151> 2015-08-14
<160> 13
<170> PatentIn version 3.5
<210> 1
   <211> 50
   <212> DNA
   <213> Artificial Sequence
<220>
   <221> misc_feature
   <222> (1)..(50)
   <223> synthetic primer
<400> 1
   tttttttttt tttttttttt tttttttttt tttttttttt tttttttttt 50
<210> 2
   <211> 60
   <212> DNA
   <213> Artificial Sequence
<220>
   <221> misc_feature
   <222> (1)..(60)
   <223> synthetic primer
<400> 2
   tttttttttt caggatgcag aggaagatga taaaacgccg cagacacatc cagcgataac 60
<210> 3
   <211> 50
   <212> DNA
   <213> Artificial Sequence
<220>
   <221> misc_feature
   <222> (1)..(50)
   <223> synthetic primer
<400> 3
   caggatgcag aggaagatga taaaacgccg cagacacatc cagcgataac 50
<210> 4
   <211> 30
   <212> DNA
   <213> Artificial Sequence
<220>
   <221> misc_feature
   <222> (1)..(30)
   <223> synthetic primer
<400> 4
   tttttttttt cagacacatc cagcgataac 30
<210> 5
   <211> 20
   <212> DNA
   <213> Artificial Sequence
<220>
   <221> misc_feature
   <222> (1)..(20)
   <223> synthetic primer
<400> 5
   cagacacatc cagcgataac 20
<210> 6
   <211> 40
   <212> DNA
   <213> Artificial Sequence
<220>
   <221> misc_feature
   <222> (1)..(40)
   <223> synthetic primer
<400> 6
   tttttttttt taaaacgccg cagacacatc cagcgataac 40
<210> 7
   <211> 29
   <212> DNA
   <213> Artificial Sequence
<220>
   <221> misc_feature
   <222> (1)..(29)
   <223> synthetic primer
<400> 7
   aaaacgccgc agacacatcc agcgataac 29
<210> 8
   <211> 50
   <212> DNA
   <213> Artificial Sequence
<220>
   <221> misc_feature
   <222> (1)..(50)
   <223> synthetic primer
<400> 8
   tttttttttt aggaagatga taaaacgccg cagacacatc cagcgataac 50
<210> 9
   <211> 40
   <212> DNA
   <213> Artificial Sequence
<220>
   <221> misc_feature
   <222> (1)..(40)
   <223> synthetic primer
<400> 9
   aggaagatga taaaacgccg cagacacatc cagcgataac 40
<210> 10
   <211> 40
   <212> DNA
   <213> Artificial Sequence
<220>
   <221> misc_feature
   <222> (1)..(40)
   <223> synthetic primer
<400> 10
   tttttttttt tttttttttt cagacacatc cagcgataac 40
<210> 11
   <211> 20
   <212> DNA
   <213> Artificial Sequence
<220>
   <221> misc_feature
   <222> (1)..(20)
   <223> synthetic primer
<400> 11
   cagacacatc cagcgataac 20
<210> 12
   <211> 70
   <212> DNA
   <213> Artificial Sequence
<220>
   <221> misc_feature
   <222> (1)..(70)
   <223> synthetic primer
<400> 12
   tttttttttt tttttttttt caggatgcag aggaagatga taaaacgccg cagacacatc 60
   cagcgataac 70
<210> 13
   <211> 50
   <212> DNA
   <213> Artificial Sequence
<220>
   <221> misc_feature
   <222> (1)..(50)
   <223> synthetic primer
<400> 13
   caggatgcag aggaagatga taaaacgccg cagacacatc cagcgataac 50

## Description

This application claims the benefit of priority from the Chinese Patent Application No. 201510501300.2 filed on August 14, 2015.

### FIELD

The present disclosure relates to the field of molecular biology, and in particular to a single molecule sequencing method and apparatus, .

### BACKGROUND

Second-generation sequencing technology is widely used in many fields, but its inherent defect such as sequencing error or bias, etc. in PCR amplification process began to highlight.

The new third-generation sequencing technology, which is characterized by sequencing technology directly directing to a single nucleic acid molecule (SMS), is emerging for overcoming the defect of the second-generation sequencing technology and is attracting more and more attention.

Helicos Corporation launched a single-molecule sequencing platform in 2008. The principle is to detect a single base fluorescence signal to achieve sequencing-by-synthesis. Specifically, the DNA to be tested is interrupted by about 200 bp before sequencing, and 40 bp of poly (A) tail with a fluorescent label is added to the 3 'end of the fragment. The library is annealed to form a single strand, which is bound to oligo dT (40 nt) probes immobilized on a chip. The company's academic paper, published in 2012, further improves the technology by directly sequencing individual DNA molecules with fluorescence but without polyA tail modification through hybridizing with specific probes. The results show the potential application of this technology, especially in clinical application. WO 2009/097626 (by Helicos Bisosciences Corp.) describes the use of a labelled target nucleic acid which is immobilized to a solid support via a captured primer for carrying out a sequencing by synthesis reaction using labelled nucleotides. But Helicos Corporation's product has not been recognized by the market due to its high price and other characteristics, therefore, the company declared bankruptcy by the end of 2011.

The single molecule sequencing technology requires further development and improvement.

### SUMMARY

The invention is defined by the appended claims.

Embodiments of the present disclosure provide a single molecule sequencing method and a single molecule sequencing apparatus.

The present invention relates to a single molecule sequencing method comprising:
(i) combining a template nucleic acid having a first optical detection label at the 5' and/or the 3'end of the template nucleic acid with a primer to obtain a first complex, wherein the primer being attached to a surface of a substrate;
(ii) imaging the first optical detection label of the first complex to obtain a first image;
(iii) mixing the first complex, polymerase, and one or more nucleotides with a second optical detection label, and thus adding the one or more of nucleotides with a second optical detection label to the first complex by polymerization reaction, thereby obtaining an extension product, wherein the nucleotides with a second optical detection label comprises a second optical detection label, a cleavable group and a nucleotide that are sequentially connected,
   wherein either (a) the nucleotide(s) with a second optical detection label is/are a monochromatic reversible terminator, wherein the monochromatic reversible terminator is any one of A, T, C, and G with the same fluorescent label and only one kind of the nucleotides with a second optical detection label is added or (b) the nucleotides with a second optical detection label are a multicolor reversible terminators, wherein the multicolor reversible terminator are at least two of A, T, C, and G with different fluorescent labels, and two or more kinds of the nucleotides with a second optical detection label are added simultaneously;
(iv) imaging the second optical detection label of the extension product to obtain a second image;
(v) removing the cleavable group from the extension product to obtain a second complex;
(vi) repeating a cycle of reaction comprising steps (ii) to (v) one or more times, wherein the second complex of (v) of the previous cycle of reaction becomes the first complex of (ii) of the next cycle of reaction; and
(vii) performing a first correction based on the first image and the second image of a same cycle of reaction, thereby correcting the positional deviation drift of the second image, and determining the template nucleic acid sequence by successively superimposing the second images after the first correction.

The present disclosure provides a single molecule sequencing method comprising: (i) combining a template nucleic acid having a first optical detection label at the end with a primer to obtain a first complex, wherein the primer being attached to a surface of a substrate; (ii) imaging the first complex to obtain a first image; (iii) mixing the first complex, polymerase, and one or more nucleotide with a second optical detection label to perform a polymerization reaction, obtaining an extension product, wherein the nucleotide with the second optical detection label comprises a second optical detection label, a cleavable group and a nucleotide that are sequentially connected; (iv) imaging the extension product to obtain a second image; (v) removing the cleavable group from the extension product to obtain a second complex; (vi) replacing the first complex with the second complex, and repeating the above steps (ii) to (v) once or more times to determine the template nucleic acid sequence.

The present disclosure provides a single molecule sequencing method, in particular comprising: (i) hybridizing a template nucleic acid having an optical detection label modified at the end with a primer attached to a surface of a substrate to form a hybridized primer-template nucleic acid complex; (ii) imaging the primer-template nucleic acid complex; (iii) mixing the primer-template nucleic acid complex, polymerase, and one or more of nucleotides with optical detection label, and thus the one or more of nucleotides with optical detection label are added to the 3' end of the primer by polymerase reaction to obtain an extension product; (iv) imaging the extended primer-template nucleic acid complex, and identifying the nucleotides to be tested on the template nucleic acid by combining the image of the primer-template nucleic acid complex in step (ii); (v) removing the cleavable groups of the nucleotides with the optical detection label on the extension product: (vi) repeating the step (ii) to (v) one or more times to identify one or more nucleotides in the template nucleic acid.

The above sequencing method achieves SMTS real-time sequencing by repeating of extension reaction, imaging detection and excision of the optical detection label molecules.

Unless otherwise specified, the "nucleic acid to be tested" and "template nucleic acid" of the present disclosure are interchangeable.

The term "first complex" is used to refer to a "primer-template nucleic acid complex" prior to the extension reaction, and the "second complex" is a complex generated by polymerization/ extension reaction to bind "nucleotide with optical detection label" to obtain an extension product and then the "optical detection label" is removed. That is, the "second complex" is a "primer-template nucleic acid complex" after removal of the cleavable groups of nucleotides with an optical detection label on the extension product. Mentioned herein, the "primer-template nucleic acid complex prior to the extension reaction" is equivalent to the "first complex"; the "primer-template nucleic acid complex after removal of the cleavable groups of nucleotides with an optical detection label on the extension product" is equivalent to the "second complex".

In the embodiments of the present disclosure, the state or manner in which the primer (probe) is attached to the surface of the substrate is not particularly limited. It is common in the art. Optionally, the primer is immobilized on the substrate surface by conventional chemical bonds or physical adsorption in the art. Optionally, the 5 'end of the primer is attached to the substrate surface in such a way that the 5' end of the polyT is attached to the substrate surface.

In an embodiment of the present disclosure, in step (i), at least part of the sequence of the primer is a sequence that is complementary to at least part of the template nucleic acid. The template nucleic acid binds to the primer by base pairing, thereby binding to the substrate.

In yet another embodiment of the present disclosure, in step (i), the primer is a sequence with 10-30 bp of polyTat the 5 'end, at least part of the primer is complementary to at least part of the template nucleic acid.

In another embodiment of the present disclosure, in step (i), the primer is a sequence with10-30 bp of polyT and an alkyl chain sequentially at the 5 'end, at least part of the primer is complementary to at least part of the template nucleic acid. Specifically, the primer is attached to the surface of the substrate at the 5 'end in a way an alkyl chain (e.g., -(CH2)6-) is attached to the epoxy group on the surface of the substrate by an amino group.

In the embodiments of the present disclosure, the length of the alkyl chain is not particularly limited. The alkyl chain is -(CH2)n-, where n is a natural number. In a specific example, n is 6.

In an embodiment of the present disclosure, the primer is attached through an amino group modified at the 5 'end to a substrate whose surface is modified with an epoxy group, including but not limited to a glass substrate, a quartz substrate, and the like. In an embodiment of the present disclosure, the step of attaching the 5 'end of the primer with the optical detection label to the surface of the substrate comprises: a) immersing the epoxy-modified glass substrate in a fixative containing 0.4-3.2nM primer for 45 min to 120 min, and then washing the substrate; b) immersing the substrate in a phosphate passivation solution, shaking, for example, for 10-15 hours; washing the substrate to obtain a substrate on which the primer is immobilized on the surface.

Further, in step a, the fixative is a 0.02 to 0.3 M K2HPO4 solution.

Further, in step a, the concentration of the primer in the fixative is preferably 0.8 to 3.2 nM, more preferably 0.8 to 1.6 nM.

Further, in step a, the substrate is washed with 3x SSC + 0.1% Triton, 3x SSC, 150 mM K2HPO4 pH = 8.5.

Further, in step b, the condition of shaking is shaking on a shaker, preferably 40-80 rpm.

Further, in step b, the phosphate passivation solution is a K2HPO4 solution having a pH of 9.0, 0.2-1 M, preferably 0.2-0.8 M.

Unless otherwise specified, the description of the optical detection label in the embodiments of the present disclosure is applicable to both the first optical detection label and the second optical detection label.

The 3 'end and/or the 5 'end of the template nucleic acid carry an optical detection label.

In an embodiment of the present disclosure, in step (i), the first optical detection label is a non-light breakable label.

In an embodiment of the present disclosure, the optical detection label is a fluorescent label. The fluorescent label is selected from one or more of fluorescein, rhodamine, cyanine, Cy5, Cy3.

The nucleotides with a second optical detection label are monochromatic reversible terminator. The monochromatic reversible terminator is any one of A, T, C, and G with the same fluorescent label; only one kind of nucleotides with fluorescent labels is added for each base extension reaction, or
the nucleotides with a second optical detection label are multicolor reversible terminators. The multicolor reversible terminator is at least two of A, T, C, and G with different fluorescent labels, and two or more kinds of nucleotides with fluorescent labels are added simultaneously for each base extension reaction.

The method of imaging the primer-template nucleic acid complex is not particularly limited. In an embodiment of the present disclosure, step ii comprises: imaging the first complex with different light sources to obtain a plurality of first images.

In an embodiment of the present disclosure, in step (ii), the step of imaging the first complex comprises: in each extension reaction, the first complex of the same site is imaged at different time points before and after the extension reaction.

In an embodiment of the present disclosure, in step (ii), the step of imaging the primer-template nucleic acid complex comprises: in each extension reaction, the primer-template nucleic acid complex at the same site is imaged at different time points before and after the extension reaction.

In an embodiment of the present disclosure, The apparatus for imaging the primer-template nucleic acid complex is not particularly limited. For example, in an embodiment of the present disclosure, a total internal reflection fluorescence imaging system provided in CN201510562591.6 can be used to excite fluorescence and acquire optical images.

In an embodiment of the present disclosure, in the step (ii) prior to the extension reaction of step (iii), the optical image is collected by a total internal reflection microscope (TIRF) to precisely locate the position of the first complex.

In an embodiment of the present disclosure, in the step (ii) prior to the extension reaction of step (iii), the optical image is collected by a total internal reflection microscope (TIRF) to precisely locate the position of the primer-template nucleic acid complex.

Analytical apparatus for positioning primers and / or template nucleic acids that are labeled with fluorescent molecules before and after extension are not particularly limited. For example, an optical image (in particular, an optical image acquired by TIRF) can be analyzed using a single molecule localization method or apparatus to accurately obtain a single - molecule two - dimensional position coordinates of the first complex or the primer-template nucleic acid complex labeled with a fluorescent molecule.

In an embodiment of the present disclosure, an optical image (in particular, an optical image acquired by TIRF) may be analyzed by using a single molecule localization method provided in CN201510483207 to accurately obtain a single - molecule two - dimensional position coordinates of the first complex or the primer-template nucleic acid complex labeled with a fluorescent molecule.

The polymerase used for the extension reaction is not particularly limited. In an embodiment of the present disclosure, the polymerase is selected from the group consisting of reverse transcriptase, terminal transferase or DNA polymerase.

In an embodiment of the present disclosure, step (iii) further comprises washing the obtained extension product.

The cleavable groups of nucleotides are not particularly limited. In an embodiment of the present disclosure, in step (iii), the cleavable group of nucleotides is a photo-cleavable group, a chemically-cleavable group or an enzyme-catalyzed cleavable group. By removing a cleavable group of a nucleotide, the optical detection label on the nucleotide is removed.

In an embodiment of the present disclosure, step (v) further comprises washing the extension product after removal of the optical detection label.

In an embodiment of the present disclosure, the method further comprises sequencing multiple target nucleic acids simultaneously.

In an embodiment of the present disclosure, step iv comprises imaging the extension product with different light sources to obtain a plurality of second images.

In another embodiment of the present disclosure, in step (iv), the step of imaging the extended first complex comprises: at the same time point after the extension reaction, the template nucleic acid in the first complex and the nucleotides with optical detection labels bound to the 3 'end of the primer chain in this extension reaction are imaged.

In an embodiment of the present disclosure, in step (iv), the step of imaging the extended primer-template nucleic acid complex comprises: at the same time point after the extension reaction, the template nucleic acid in the primer-template nucleic acid complex and the nucleotides with optical detection labels bound to the 3 'end of the primer chain in the extension reaction are imaged.

In an embodiment of the present disclosure, the step of (iv) comprises identifying the nucleotide species introduced in step (iii) by collecting an optical image through a total internal reflection microscope (TIRF).

The apparatus for imaging the primer-template nucleic acid complex is not particularly limited. Total internal reflection microscopy system (TIRF) can be used to enhance the signal to noise ratio. According to an embodiment of the present disclosure, the template nucleic acid and the fluorescently labeled nucleotides are photographed after each extension reaction of step (iii), respectively; by two times of imaging of the same coordinate field, the slight deviation due to the slight movement of the stage or sample drift while imaging can be corrected.

In an embodiment of the present disclosure, in step (iv), the step of imaging the extended primer-template nucleic acid complex, and identifying the nucleotides to be tested on the template nucleic acid by combining the image of the primer-template nucleic acid complex in step (ii), comprises: imaging the extended primer-template nucleic acid complex, and performing image correction by combining the image of the primer-template nucleic acid complex in step (ii), to identify the nucleotide sequence to be tested on the template nucleic acid.

The apparatus for performing image correction is not particularly limited. For example, in an embodiment of the present disclosure, a single molecule image correction apparatus provided in CN201510500962.8 may be used to perform single molecule image correction of an optical image acquired at different times (in particular, an optical image acquired by TIRF). In an embodiment of the present disclosure, a single molecule image correction method provided in CN201510501154.3 may be used to perform single molecule image correction of an optical image acquired at different times (in particular, an optical image acquired by TIRF).

Unless otherwise specified, "image comparison and correction" is equivalent to "image correction".

The single molecule sequencing method further comprises determining a template nucleic acid sequence based on the first image and the second image.

In an embodiment of the present disclosure, determining a template nucleic acid sequence based on the first image and the second image comprises: comparing the difference between the first image and / or the second image, and performing a first correction and / or a second correction on the first image and / or the second image, to obtain a corrected first image and / or a corrected second image. The correction processing of the image can at least partially correct the positional deviation of the fluorescence spot at the time of image acquisition due to the drift of the stage in the reaction or the drift of the reagent washing in the reaction, which facilitates the base recognition based on the image.

Further, the first correction is performed based on the first image and the second image in the same cycle of reaction, and the second correction is performed based on between the first images or between the second images in the adjacent cycle of reaction, wherein from (ii) to (v) is defined as a cycle of reaction. The images in the adjacent cycle of reaction may be a plurality of first images or a plurality of second images in adjacent two cycles, adjacent three cycles, adjacent five cycles, or adjacent ten cycles of reactions.

In an embodiment of the present disclosure, the step of performing image correction includes one or both of a first correction process and a second correction process, wherein the first correction process comprises: performing image comparison and correction for the images of the first complex of the same site at different time points before and after the extension reaction; the second correction process comprises: performing image correction for the images of the template nucleic acid of the same site after a certain cycle of extension reaction and the images of the nucleotide having an optical detection label bound to the site in the extension reaction.

In an embodiment of the present disclosure, the method of comparison and correction of a plurality of single-molecule images taken at the same site at different time points, comprises: 1, first reading a single molecule imaging picture at a first time point and then reading a single molecule imaging picture at a second time point, both are stored as a uintl6 format matrix in the matlab; 2, performing a two-dimensional Fourier transform for the single molecule imaging picture matrix of the first time point, and saving the transformed matrix *fft_ref*; 3 , performing a two-dimensional Fourier transform for the single molecule imaging picture matrix of the second time point, and saving the transformed matrix *fft_frame*; acquiring the convolution *prod* =*fft_ref.*conj(fft_frαme)* of the matrixes after the image Fourier transform at both time points; 5, performing a two-dimensional Fourier inverse transform for the *prod* matrix to obtain the matrix cc *= ifft2 (prod);* 6, performing a *fftshift* transform for the cc matrix, finding the maximum coordinates of the matrix after transformation, and subtracting a half of the original image size, and therefore obtaining the image offset of different time points; 7, and finally according to the offset, correcting the offset for the second time point of the picture with *circshift* function.

It is to be understood that in the embodiments of the present disclosure, each cycle of extension reactions involves imaging; the correction process involved in each cycle of imaging, the correction can be carried out in each cycle of reactions, or after multiple cycles of reactions. According to an embodiment of the present disclosure, all of the imaged pictures are subjected to correction process after the end of all extension reactions.

In an embodiment of the present disclosure, the single molecule sequencing method further comprises: obtaining a template nucleic acid; treating the template nucleic acid to obtain a template nucleic acid with a first optical detection label at its end. The template nucleic acids are DNA and / or RNA.

In an embodiment of the present disclosure, the single molecule sequencing method further comprises subjecting the sequencing results to bioinformatics analysis.

The present disclosure provides a single molecule sequencing apparatus capable of implementing the sequencing method in any of the embodiments of the present disclosure described above, the apparatus comprises: a combining module, for combining a template nucleic acid having a first optical detection label at the end with a primer to obtain a first complex, wherein the primer being attached to a surface of a substrate; a first imaging module, for imaging the first complex from the combining module to obtain a first image; a synthesizing module, for mixing the first complex from the combining module, polymerase, and one or more of nucleotides with a second optical detection label, and adding the one or more of nucleotides with the second optical detection label to the first complex by polymerase reaction, therefore obtaining an extension product, wherein the nucleotides with the second optical detection label comprises a second optical detection label, a cleavable group and a nucleotide that are sequentially connected; a second imaging module, for imaging the extension product from the synthesizing module to obtain a second image; a cleaving module, for removing the cleavable group from the extension product from the synthesizing module to obtain a second complex; an iterating module, for replacing the first complex from the combining module with the second complex from the cleaving module, and entering in turn the second imaging module, the synthesizing module, the second imaging module and the cleaving module one or more times to determine the template nucleic acid sequence.

The present invention relates to a single molecule sequencing apparatus comprising:
a combining module, for combining a template nucleic acid having a first optical detection label at the 5' and/or the 3'end with a primer to obtain a first complex, wherein the primer being attached to a surface of a substrate;
a first imaging module, for imaging the first complex from the combining module to obtain a first image;
a synthesizing module, for mixing the first complex from the combining module, polymerase, and one or more of nucleotides with a second optical detection label, and thus adding the one or more of nucleotides with the second optical detection label to the first complex by polymerase reaction, obtaining an extension product, wherein the nucleotides with the second optical detection label comprises a second optical detection label, a cleavable group and a nucleotide that are sequentially connected;
a second imaging module, for imaging the extension product from the synthesizing module to obtain a second image;
a cleaving module, for removing the cleavable group from the extension product from the synthesizing module to obtain a second complex;
an iterating module, for replacing the first complex from the combining module with the second complex from the cleaving module, and entering in turn the first imaging module, the synthesizing module, the second imaging module and the cleaving module one or more times to determine the template nucleic acid sequence; and
an image processing module, for determining the template nucleic acid sequence by comparing the difference between the first image and the second image, and performing a first correction based on the first image and the second image of the same cycle of reaction based on the positional drift to obtain a corrected second image.

It will be understood by those skilled in the art that the above-described apparatus of this aspect of the disclosure can be used to implement the single molecule sequencing method described in any of the embodiments of the present disclosure by making the functional module have a corresponding function or by adding a new functional module or sub-module, the above description of the advantages or technical characteristics of the sequencing method according to any one of the embodiments of the present disclosure is also applicable to the apparatus of this aspect of the present disclosure.

The present disclosure provides a single molecule sequencing apparatus comprising: a chip chamber for providing a reaction chamber for a sequencing reaction; a substrate disposed within the chip chamber for immobilizing a primer and binding a nucleic acid to be tested; a flow path system connected to the chip chamber for controllable manipulation of the treatment reagent in and out of the chip chamber where the substrate is located; a temperature control system connected to the chip chamber for regulating and maintaining the temperature in the chip chamber; an optical system, which is connected to the chip chamber and comprises a laser light source for exciting the fluorescent label in the chip chamber to emit fluorescence; a detector component connected to the chip chamber for detecting and recording the fluorescent signal emitted from the fluorescent label in the chip chamber; a computer having a control system and an image processing unit, wherein the image processing unit is used to obtain the positioning result of the primer-nucleic acid to be tested complex before and after the extension reaction in the sequencing reaction in the chip chamber, and to perform image comparison and correction for the obtained positioning result, and thus identifying the sequence of the nucleic acid to be tested.

In an embodiment of the present disclosure, the image processing unit is also used for image comparison and correction for the image of the nucleic acid to be tested after the extension reaction wherein the nucleic acid to be tested is the nucleic acid before and after the extension reaction in the process of the sequencing reaction in the chip chamber, and for the image of the nucleotide with the optical detection label bound to the site in the extension reaction.

Wherein fluid control device for the gene sequencing is one of the key modules of the single molecule apparatus, and the gene chip in the fluid control device for the single molecule gene sequencing is the core component for biochemical reaction and optical imaging, and the fluid control device for the gene sequencing is usually a plurality of fluid delivery devices, and the fluid delivery devices have a function of delivering various reaction reagents to the gene chip, controlling the flow direction and rate of the reagents, controlling the mixing of the reagents, and exporting the waste liquid, and the like.

In the present disclosure, the flow path system may deliver a variety of reaction reagents to the chip chamber in which the substrate (e.g., a gene chip) is located by the fluid control device for gene sequencing, and control the flow direction and rate of the reagents, control the mixing of the reagents, and export the waste liquid, and the like, by the computer control system.

In the present disclosure, the optical system and the detector component form a fluorescence imaging system, optionally, the fluorescence imaging system may use a total internal reflection fluorescence imaging system to excite the fluorescence and collect the optical image.

In the present disclosure, the computer controlled system includes a flow path system, a temperature control system, an optical and detection system. Optionally, it also includes a data analysis software.

In an embodiment of the present disclosure, the image processing unit includes a single molecule positioning module and a single molecule image correction module.

In the embodiments of the present disclosure, the single molecule positioning module is used to position the primers and / or template nucleic acids that are labeled with fluorescent molecules before and after extension, and the single molecule positioning module is not particularly limited. For example, an optical image, such as an optical image acquired by TIRF, can be analyzed using a single molecule positioning device to accurately obtain the two-dimensional position coordinates of the primer-template nucleic acid complex labeled with a fluorescent molecule.

In the embodiments of the present disclosure, the single molecule image correction module is used for image comparison and correction for the positioning result of the primer / nucleic acid to be test complex before and after the extension reaction in the sequencing reaction in the chip chamber to identify the sequence of the nucleic acid to be tested. There is no particular restriction on how to perform the image correction by the single molecule image correction module.

The present disclosure provides a sequencing system comprising a sequencing apparatus or a single molecule sequencing apparatus in any of the above embodiments. Optionally, it also includes a data analysis software.

The present disclosure provides a sequencing kit comprising a substrate and a primer in a sequencing method in any of the above embodiments.

In the present disclosure, the sequencing kit further comprises a reagent required to achieve the method in any of the above embodiments. In particular, it may include at least one of a fixing reaction reagent, an extension reaction reagent, an imaging reagent, and a reagent for excising an optical detection label molecule.

It is to be understood that the single molecule sequencing kit may also include buffers or other sequencing essential reagents. The substrate, the fixing reaction reagent, the extension reaction reagent, the imaging reagent, and the reagent for excising an optical detection label molecule are not particularly limited. It is generally common in the art. For example, a person skilled in the art, can prepare the fixing reaction reagent, the extension reaction reagent, and the reagent for excising an optical detection label molecule, and other buffers used in different process, according to a specific need.

In the determination of a DNA sequence, the sequencing solution provided by the embodiments of the present disclosure is achieved by randomly immobilizing a primer on a substrate; hybridizing a small fragment DNA template with an optical detection label at the end with the immobilized primer and precisely positioning; accurately positioning the location of the template after hybridization by collecting the optical image using a total internal reflection microscope (TIRF); gradually adding a mixture of a monochromatic or multicolor terminal terminator with a cleavable optical detection label, and polymerase, to incubate, wash, perform optical imaging, record the reaction point of the reaction; then adding a reagent to cleave the optical detection label at the extension point, washing, capping, ready to the extension reaction for the next nucleotide. The single molecule target real-time sequencing can be achieved by repeating of extension reaction, image detection and excision of fluorescent molecules.

### BRIEF DESCRIPTION OF THE DRAWINGS

Fig. 1 is a schematic diagram of a single molecule sequencing apparatus 01 provided by the present disclosure;
Fig. 2 is a schematic diagram of a single molecule sequencing principle provided by an embodiment of the present disclosure;
Fig. 3 is a schematic diagram of a principle for immobilizing a primer according to an embodiment of the present disclosure;
Fig.s 4-7 are the results of a nucleic acid chip immobilization reaction provided in the examples of the present disclosure.

### DETAILED DESCRIPTION

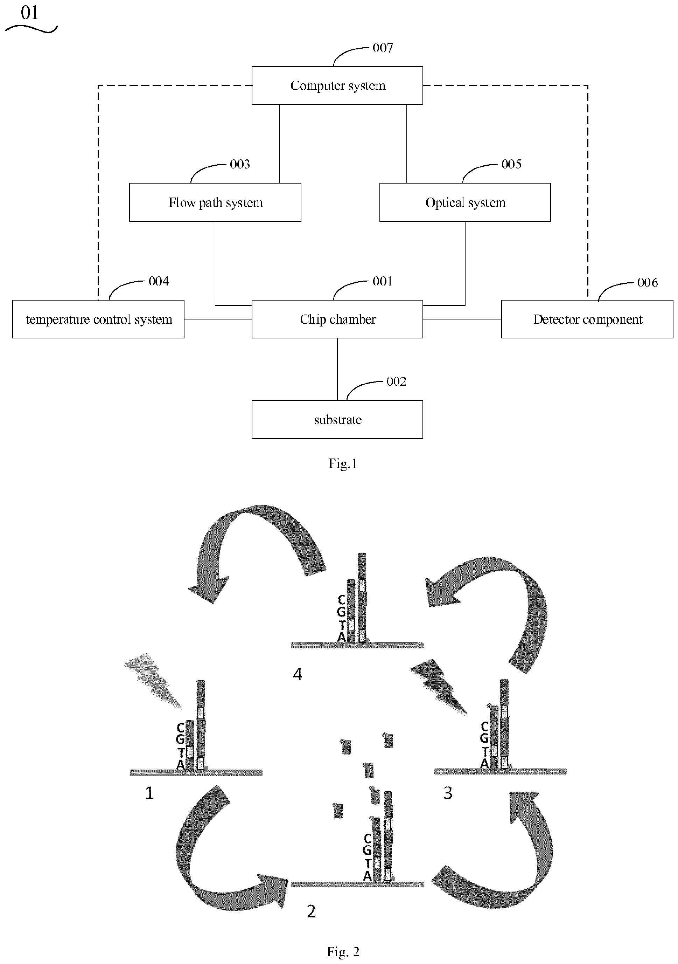

Fig. 1 is a schematic diagram of a single molecule sequencing apparatus 01 provided in the present disclosure. The single molecule sequencing system provided by the present disclosure comprises the single molecule sequencing apparatus 01 provided by the present disclosure.

As shown in Fig. 1, the single molecule sequencing apparatus 01 can be used to image a planar substrate on which a primer is bound. When used in sequencing, the apparatus may include a chip chamber 001 for providing a reaction chamber for sequencing reaction, so that the nucleic acid to be tested and the reagents delivered by a flow path system can generate a sequencing reaction, a substrate 002 disposed in the chip chamber, used for binding one or more primers; a flow path system 003 connected to the chip chamber for controllably manipulating various reagents (for example, buffers, enzymes, fluorescently labeled nucleotides, etc.) into or out of the chip chamber in which the substrate is located; a temperature control system 004 connected to the chip chamber for regulating and maintaining the temperature in the chip chamber; an optical system 005 connected to the chip chamber including a laser light source (e.g., one or more lasers), the optical system for exciting the fluorescent label in the chip chamber to emit fluorescence; a detector component 006 (e.g., an EMCCD camera, etc.) connected to the chip chamber for detecting and recording the fluorescent signal emitted from the fluorescent label in the chip chamber; a computer 007 having various components for controlling systems (such as a flow path system, a temperature control system, an optical and detection system, an image system, and a data analysis software, wherein the computer 007 and the flow path system 003, the temperature control system 004, the optical system 005 and the detector component 006 are communication connection). Wherein, the image system is used to obtain the positioning result of the primer-nucleic acid to be tested complex before and after the extension reaction in the sequencing reaction in the chip chamber, and to perform image comparison and correction for the obtained positioning result, and thus identifying the sequence of the nucleic acid to be tested.

In an embodiment of the present disclosure, the single molecule sequencing is performed using the above described single molecule sequencing apparatus, comprising the steps of: covalently bonding the template / primer double strand to the epoxy group on the surface of the substrate; performing sequencing by synthesis reaction and optical detection the nucleotides incorporated.

It will be appreciated by those skilled in the art that the sequencing method of the present disclosure can be used for partial sequencing, DNA fingerprinting, polymorphism identification, such as detection of single nucleotide polymorphisms (SNPs), and genetic cancer research applications. The method of the present disclosure can also be used for RNA sequence sequencing to determine alternative splice sites, copy numbers, detect gene expression, and identify RNA molecules present in low numbers in unknown cells. The method of the present disclosure can also be used to determine which sequence transcription to annotate the genome, to determine phylogenetic relationships, to elucidate cell differentiation, and promote tissue engineering.

For different medical applications, the required nucleic acid template is extracted and pretreated by a corresponding method. In an embodiment of the disclosure, the nucleic acid template molecule comprises deoxyribonucleic acid (DNA) and / or ribonucleic acid (RNA). The nucleic acid template molecules can be isolated from biological samples containing various other components, such as proteins, lipids, and non-template nucleic acids. The nucleic acid template molecules can be obtained from animals, plants, bacteria, fungi, or any other cellular organism. The biological sample of the present disclosure includes a virus (DNA or RNA virus). Nucleic acid template molecules can be obtained directly from biological organisms, such as from blood, urine, cerebrospinal fluid, semen, saliva, sputum, feces or other tissue. Any tissue or body fluid sample can be used as a source of nucleic acid used in the present disclosure. Nucleic acid template molecules can also be isolated from cultured cells, such as primary cell cultures or cell lines. The sample may also be total RNA or genomic DNA extracted from a biological sample.

The nucleic acid is usually extracted from the biological sample and is interrupted to produce a suitable fragment for analysis. In general, the nucleic acid is randomly interrupted by about 200 bp fragment. In an embodiment, the nucleic acid extracted from the biological sample is interrupted by sonication. In general, various techniques for extracting nucleic acid from biological samples are conventional techniques within the art, such as Maniatis et al., Manual of Molecular Cloning Laboratory, Cold Spring Harbor, NY, 280-281 (1982). In general, the length of a single nucleic acid template molecule may be from about 5 bp to about 20 kb. Nucleic acid molecules can be single-stranded, double-stranded, or single-stranded regions of double-strand (e.g., stem and loop structures).

Primers (complementary to partial sequences of the nucleic acid fragments to be tested) cover the desired sequencing gene fragments as much as possible, stably and efficiently capturing the targeted DNA fragments, such as the sequencing of the HBV virus, primers covering all gene mutation sites and drug-resistent sites are designed as much as possible. The primers are generally immobilized on an optically transparent, modified substrate. The substrate surface needs to be chemically modified so that the primers are immobilized on the substrate surface by chemical bonds or physical adsorption. The substrate is any suitable carrier with low natural fluorescence or substantially no fluorescence.

In some embodiments, the substrate may be two- or three-dimensional, and may include a flat surface (e.g., a glass slide), or may be other shapes. The substrate may include glass (e.g., controllable porosity glass (CPG)), quartz, plastic (e.g., polystyrene, not limited to low crosslinked and highly crosslinked polystyrene), polycarbonate, polypropylene and polymethymethacrylate, acrylic acid copolymers, polyamides, silicones, metals (e.g., alkanethiolate-derived gold), cellulose, nylon, latex, dextran, gel matrix (e.g., silicone), polyacrolein, or composites.

The three-dimensional substrates, for example, pellets, microparticles, beads, films, slides, slabs, micromachined chips, tubes (e.g., capillaries), micropores, microfluidic devices, channels, filters, or any other structures suitable for anchoring nucleic acids. The substrate may comprise a planar array or matrix having a primer region, such as a nucleoside-derived CPG and a polystyrenesubstrate; a derived magnetic substrate or the like.

Primers can be immobilized on the substrate surface by conventional chemical or physical adsorption in the art and can be immobilized on the substrate surface either directly or indirectly (e.g., via biotin). In an specific example, the inventors use a low fluorescent glass surface treated with epoxysilane, the epoxy bond on its surface can be chemically bonded to the amino group at the end of the primer, and the primer 5 'end includes polythymidine (poly dT). In some embodiments, the 5 'end of the primer is linked to the 3 'end of the polythymidine (poly dT), and the 5 'end of the poly dT oligonucleic acid chain is bonded to the epoxy bond of the substrate surface by an amino group. In an embodiment, the 5 'end of the primer molecule is sequentially linked to a polythymidine (poly dT) chain, an alkyl chain (e.g., -(CH₂)₆-) and a terminal amino group, wherein the 3 'end of the poly dToligonucleic acid chain is linked to the 5' end of the primer molecule, and the 5 'end of the poly dT oligonucleotide chain is linked to the alkyl chain, and the alkyl chain is bonded to the epoxy bond of the surface of the substrate through an amino group.

The fixed primer density requirement is such that the density is higher as much as possible in ensuring the dispersion of the single molecule level, thus ensuring the highest possible sequencing flux. In an embodiment, the substrate may employ other treatments within the art to improve nucleic acid attachment efficiency. In other embodiments, the substrate may be processed to reduce background noise. The epoxide used to modify the substrate may also be a derivative of the epoxide.

In a specific example, after the primers are immobilized on the substrate, a chemical passivation process is also required to eliminate the unblocked epoxy groups and to prevent the noise generated by the nonspecific adsorption during the sequencing process. There are many ways to pass the surface passivation process. Since the fluorescently labeled base molecules in the sequencing are negatively charged, phosphates can be used to block the epoxy groups on the glass surface and produce a negative layer to reduce the negatively charged base adsorption.

In conjunction with Fig. 2, a single molecule sequencing method is provided, comprising the following steps: sample preparation, substrate surface treatment, and primer immobilization, imaging, and image processing to obtain the sequence. Primers may be oligonucleotides at the 5' end including polythymidine (poly dT).

In a specific embodiment, as shown in Fig. 2,the sequence to be tested is interrupted into a small fragment DNA template before sequencing. The end of the DNA template is labeled with an optically detectable label molecule to record and position the coordinates of the DNA template by optical microscopy. At the same time, a plurality of primers comprising poly dT at the 5' end are randomly attached to the substrate disposed in the chip chamber. The small fragment DNA template is hybridized with the immobilized primers and accurately positioned, and the optical image is collected by a total internal reflection microscope (TIRF) to accurately position the position of the hybridized small fragment DNA template. And then a mixture of nucleotides with a detection label and polymerase is added, incubate, wash, perform optical imaging, and record the site of the present reaction. Reagents are then added to remove the fluorescent molecules at the extended sites, wash, cap, and ready for the next nucleotide extension reaction. Real-time sequencing can be achieved by repeated cycles of extension reaction, imaging detection and excising the fluorescent molecules.

The polymerase used in the above sequencing method can be Klenow polymerase with reduced exonuclease activity. Nucleic acid polymerases can be used include, but are not limited to, DNA polymerases, RNA polymerases, reverse transcriptases, and / or any of the above-described polymerase mutants described in literatures or commercially available in the art.

In an embodiment of the present disclosure, the nucleotide has different light-emitting groups, and in a cycle of sequencing reaction, a variety of nucleotides can be simultaneously fed to the same reaction system.

The third generation sequencing techniques characterized with the single nucleotide sequencing achieves single nucleotide extension to improve sequencing accuracy by cyclic reversible termination (CRT) approach. That is, when a nucleotide with an inhibiting group is added to the DNA strand, the extension of the next nucleotide can be prevented; under a mild condition the repressor nucleotide can be removed so that the DNA strand continues to extend. For each addition of a nucleotide, real-time sequencing of the DNA strand can be achieved by its detection of fluorescence. Such a nucleotide with an inhibiting group is referred to as a terminator. The nucleotides with the detection label employed are monochromatic or multicolor reversible terminators with a cleavable fluorescent label. In an embodiment of the present disclosure, the reversible terminal terminator is a light-cleavable fluorescently labeled reversible terminal compound. In another embodiment of the present disclosure, the reversible terminal terminator is a fluorescently labeled reversible terminal compound that is chemically cleavable or enzyme-catalyzed cleavable.

In some embodiments, at least 3, at least 5, at least 10, at least 20, at least 30, at least 50, at least 100, at least 500, at least 1000, or at least 10,000 continuous cycling reactions are used for extending the primers hybridized specifically with a template chain. In each cycle, extending one fluorescently labeled reversible terminator, and before the subsequent cycle, the fluorescently labeled reversible terminator is removed its fluorescent label and the inhibiting group.

In general, the sequencing by synthesis requires precise temperature control and multiple chemically washing. Sequencing samples are packaged in a well-designed microfluidic control system to ensure accurate temperature and reagent flow. In order to ensure the throughput of sequencing, in an embodiment of the present disclosure, a plurality of channels may be provided. The assembled sample is finally placed on a total internal reflection optical microscope that can observe the single molecule signal. The optical signals include fluorescence, Raman, scattering, and so on. In a specific example of the disclosure, the observed optical signal is a single molecule fluorescent signal. In order to prevent the positioning signal and the sequencing signal from overlapping, different excitation signals are used respectively to stimulate the positioning fluorescent molecules and sequencing label molecules. In the process of multiple imaging, the fluorescence molecules will quench, resulting in loss of sequencing signal and short of sequencing read length, in order to improve these problems, adding imaging reagents while imaging, shortening the exposure time, reducing the intensity of excitation and other means to ensure the sequencing accuracy.

In an embodiment of the present disclosure, the single molecule sequencing method further comprises: an image acquisition process. The image acquisition uses a total internal reflection microscopy system (TIRF). In general, the single molecule signal is very weak and the TIRF system can significantly reduce the background noise and improve the signal-to-noise ratio. In an embodiment of the present disclosure, a double-color laser light path system is used to process the sample encapsulated in the microfluidic system and then photographed before the image is collected. In order to prevent the sample from drifting, the positioning information needs to be photographed before each extension reaction, and then photographed after base extension to obtain a sequencing signal.

A total internal reflection microscope (TIRF) system can be employed to perform the step of collecting an optical image, it can enhance the signal to noise ratio. According to an embodiment of the present disclosure, photographing before and after the extension reaction of step (iii), respectively; by two times of imaging of the same coordinate field, the slight deviation due to the slight movement of the stage or sample drift while imaging can be corrected.

In an embodiment of the present disclosure, the method of comparison and correction of a plurality of single-molecule images taken at the same site at different time points, comprises: 1, first reading a single molecule imaging picture at a first time point and then reading a single molecule imaging picture at a second time point, both are stored as a uintl6 format matrix in the matlab; 2, performing a two-dimensional Fourier transform for the single molecule imaging picture matrix of the first time point, and saving the transformed matrix *fft_ref*; 3 , performing a two-dimensional Fourier transform for the single molecule imaging picture matrix of the second time point, and saving the transformed matrix *fft_frame*; acquiring the convolution *prod* =*fft_ref.*conj(fft_frαme)* of the matrixes after the image Fourier transform at both time points; 5, performing a two-dimensional Fourier inverse transform for the *prod* matrix to obtain the matrix *cc* = *ifft2 (prod);* 6, performing a *fftshift* transform for the cc matrix, finding the maximum coordinates of the matrix after transformation, and subtracting a half of the original image size, and therefore obtaining the image offset of different time points; 7, and finally according to the offset, correcting the offset for the second time point of the picture with *circshift* function.

In an embodiment of the present disclosure, the single molecule sequencing method further comprises: an image processing process. In the imaging step, the image obtained by each nucleotide extension reaction may have several tens or thousands of fields of view. For image processing, it is necessary to accurately calculate the coordinate position of each reaction and record it. The images obtained during each subsequent base extension are subjected to image correction software to correct the position drift caused by chemical flushing process and sample movement. And then the images are overlapped, and the positions of the sequencing reactions are successively superimposed and calculated to obtain the base sequence at each position.

In an embodiment of the present disclosure, the single molecule sequencing method provided by the present disclosure further comprises: a bioinformatics analysis process. On the basis of the obtained base sequence, the complete sequence of the DNA fragment was finally obtained by base determination and alignment. Bioinformatics involved to complete the analysis of the biological significance of the fragment, and to help doctors determine the choice of drugs, screening of diseases.

### Examples

### Materials and reagents:

Unless otherwise specified, the reagents used in the examples are commercially available, and the databases used in the examples are disclosed online databases.

20 x SSC: Dissolving 175.3 g NaCl and 88.2 g sodium citrate in 800 ml water, adding a few drops of 10mol/L NaOH solution to adjust the pH to 7.0, adding water to 1 L, and autoclaving after packaging. SSC is the most standard imprinting and molecular hybridization solution for molecular biology. 20 × SSC is used for hybridization experiments and purifying commonly used concentrated buffer.

### DNA chip immobilization

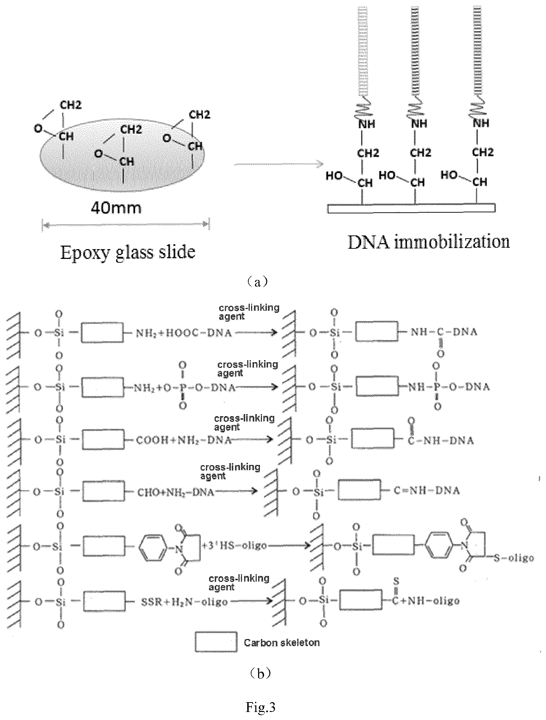

As shown in Fig. 3, Fig. 3 is a schematic diagram of a method for immobilizing a primer according to an embodiment of the present disclosure. Fig. 3 includes a-b, a is a schematic diagram for immobilizing a primer DNA on an epoxy glass slide, and b is a schematic diagram of a method for immobilizing a primer on a slide having other groups modified. The technology utilizes the advantages that the epoxy group itself is unstable and has a large tension and can react chemically with the DNA modified with -NH₂. The DNA single strand to be immobilized is immobilized on the surface of an epoxy-modified chip through a new -CH₂- NH- covalent bond. The immobilization technique is only a specific example of the present disclosure, and the optimization of the method of the present disclosure is effective for all immobilized vectors and DNA sequences. The 5 'end of the DNA sequence has a group which can react chemically with the chip, such as amino, aldehyde, carboxyl, mercapto and the like; the 3' end is modified with a single molecule fluorescent substance, such as Cy3, Cy5, for the purpose of counting the immobilized number.

Specifically, examples of the present disclosure provide a single molecule sequencing method comprising immobilizing a primer on a chip, comprising the steps of: 1) purging the chip to be immobilized with a nitrogen gas gun, the substrate surface having epoxy, the substrate slide is the epoxy-modified slide series from SCHOTT company; 2) the DNA is diluted with a fixative (0.02-0.3 M K₂HPO₄, 0.2 M in this example) to a concentration of 800-1600pM (0.8 nM in this example), the chip is immersed in a DNA- fixative for 45 min to 120 min (60 min in this example), and the chip is just completely in the fixative; 3) and then the chip is washing successively by 3x SSC +0.1% Triton, 3x SSC, 150 mM K₂HPO₄ pH = 8.5; 4) and then pH = 9.0, 0.2-1 M K₂HPO₄ (1M in this example) solution be used to immerse the chip, and at room temperature the chip is shaken at 80 rpm (r / min) for 15 hours on a shaker; 5) the chip is washed sequentially with PBS, 150 mMHepes + 150 mMNaCl, and finally the chipis washed with double distilled water; 6) The immobilized chip is photographed by fluorescence microscopy , each chip is photographed 20 consecutive fields of vision, and then counting each photo the number of single molecule fluorescent spots of the film, calculating the average of the number of fluorescent spots in each field of view.

Specifically, in order to observe the effect of the polyT of the primer tail on the immobilizing density, the following DNA is carried out the immobilization (the underlined portion is -NH₂(CH₂)₆-):
T50-Cy3 (5'→3'):
   AmMC6TTTTTTTTTTTTTTTTTTTTTTTTTTTTTTTTTTTTTTTTTTTTTTTTTT-Cy3, specifically,
   TTTTTTTTTTTTTTTTTTTTTTTTTTTTTTTTTTTTTTTTTTTTTTTTTT (SEQUENCE NO.1).
T10C50-Cy3 (5'→3'):
   AmMC6TTTTTTTTTTCAGGATGCAGAGGAAGATGATAAAACGCCGCAGACACATCCAGCGATAAC-Cy3 , specifically,
   TTTTTTTTTTCAGGATGCAGAGGAAGATGATAAAACGCCGCAGACACATCCAGCGATAAC (SEQUENCE NO.2).
C50-Cy3 (5'→3'):
   AmMC6CAGGATGCAGAGGAAGATGATAAAACGCCGCAGACACATCCAGCGATAAC-Cy3 specifically,
   CAGGATGCAGAGGAAGATGATAAAACGCCGCAGACACATCCAGCGATAAC (SEQUENCE NO.3).

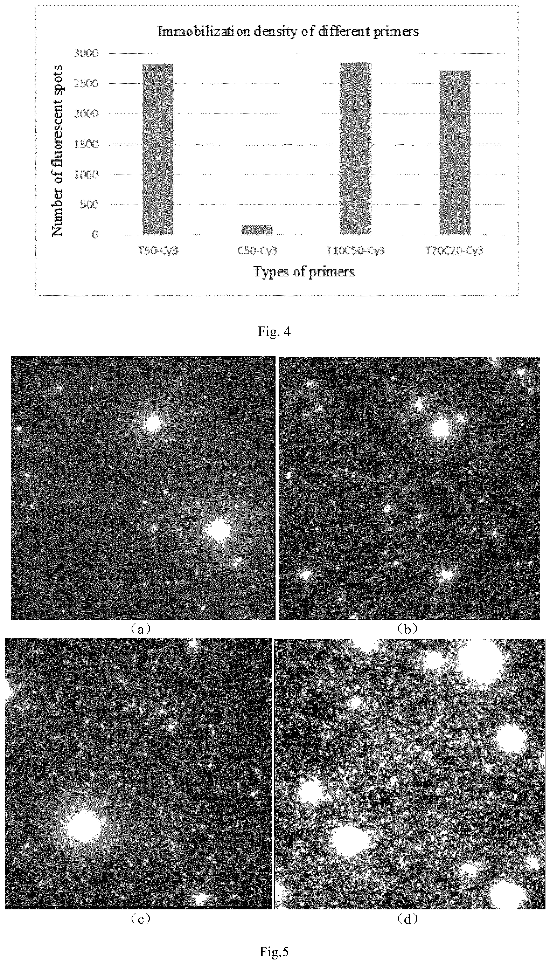

In the present example, T50-Cy3, C50-Cy3 and T10C50-Cy3 are respectively immobilized on the chip and operated in accordance with the above-described immobilization step, and repeating six times and the number of sequences immobilized are counted. The results are shown in Fig. 4. It can be seen from Fig. 4 that when the DNA is changed from T50-Cy3 to C50-Cy3, the immobilization density is reduced from 2800 to 150; when 10bp polyT is added to the 5 'end of C50, the immobilization density increases to 2800 (after subsequent experiments, 10-30 bppolyT, preferably 10-20 bppolyT can increase the immobilization density to about 2500-3200, preferably 2800), indicating that the presence of a segment of base T at the 5 'end of the DNA greatly increases the immobilization density.

Thus, 10 to 30 bp of polyT, preferably 10bp is preferably attached to the 5 'end of the primer to be immobilized.

Specifically, in order to further observe the effect of the number of polyT of the primer tail and the different primer sequences on the immobilization density, the following DNA is carried out the immobilization (the underlined portion is -NH₂(CH₂)₆-):
T10C20-Cy3 (5'→3'):
   AmMC6TTTTTTTTTTCAGACACATCCAGCGATAAC-Cy3, specifically,
   TTTTTTTTTTCAGACACATCCAGCGATAAC (SEQUENCE NO.4).
C20-Cy3 (5'→3'):
   AmMC6CAGACACATCCAGCGATAAC-Cy3, specifically,
   CAGACACATCCAGCGATAAC (SEQUENCE NO.5).
T10C30-Cy3 (5'→3'):
   AmMC6TTTTTTTTTTTAAAACGCCGCAGACACATCCAGCGATAAC-Cy3, specifically,
   TTTTTTTTTTTAAAACGCCGCAGACACATCCAGCGATAAC (SEQUENCE NO.6).
C30-Cy3 (5'→3'):
   AmMC6AAAACGCCGCAGACACATCCAGCGATAAC-Cy3, specifically,
   AAAACGCCGCAGACACATCCAGCGATAAC (SEQUENCE NO.7).
T10C40-Cy3 (5'→3'):
   AmMC6TTTTTTTTTTAGGAAGATGATAAAACGCCGCAGACACATCCAGCGATAAC-Cy3, specifically,
   TTTTTTTTTTAGGAAGATGATAAAACGCCGCAGACACATCCAGCGATAAC (SEQUENCE NO.8).
C40-Cy3 (5'→3'):
   AmMC6AGGAAGATGATAAAACGCCGCAGACACATCCAGCGATAAC-Cy3, Specifically,
AGGAAGATGATAAAACGCCGCAGACACATCCAGCGATAAC (SEQUENCE NO.9).
T20C20-Cy3 (5'→3'):
   AmMC6TTTTTTTTTTTTTTTTTTTTCAGACACATCCAGCGATAAC-Cy3, specifically,
   TTTTTTTTTTTTTTTTTTTTCAGACACATCCAGCGATAAC (SEQUENCE NO.10).
C20-Cy3 (5'→3'):
   AmMC6CAGACACATCCAGCGATAAC-Cy3, specifically,
   CAGACACATCCAGCGATAAC (SEQUENCE NO.11).
T20C50-Cy3 (5'→3'): 
AmMC6TTTTTTTTTTTTTTTTTTTCAGGATGCAGAGGAAGATGATAAAACGCCGCAGACACATCCAGCG
ATAAC-Cy3，
 specifically, TTTTTTTTTTTTTTTTTTTTCAGGATGCAGAGGAAGATGATAAAACGCCGCAGACACATCCAGCGATAAC (SEQUENCE NO.12).
C50-Cy3 (5'→3'):
   AmMC6CAGGATGCAGAGGAAGATGATAAAACGCCGCAGACACATCCAGCGATAAC-Cy3, specifically,
   CAGGATGCAGAGGAAGATGATAAAACGCCGCAGACACATCCAGCGATAAC (SEQUENCE NO.13).

The results showed, when the above DNA chip immobilization method is used for immobilization, thatsimilar results are achieved by the above DNA as T10C50-Cy3, and similar immobilization rule as "T10C50-Cy3 and C50-Cy3" : when the DNA is changed from polyT-(CH2)n-DNA-Cy3 to -(CH₂)n-DNA-Cy3, the immobilization density is reduced from 2000-3500 to about 150; When 10-30 bp polyT is added to the 5 'end of -(CH₂)n- the immobilization density is increased to 2000-3500, indicating that the presence of a segment of base T at the 5 'end of the DNA greatly increases the immobilization density. Partial experimental results in this example are shown in Fig. 3.

### Effect of Rotation Speed on Fluorescent Spot in DNA Chip Passivation

The operation is carried out according to the experimental method described in "DNA Chip Immobilization" described above, except that:
In step 1), the DNA to be immobilized is T10C50-Cy3 at a concentration of 0.4 nM, 0.8 nM, 1.6 nM, 3.2 nM, respectively. In addition, in step 4), two groups of experiments were passivated at 0 rpm / min and 80 rpm / min shaking conditions, respectively. In the example of the present disclosure, "rpm / min" and "r / min" are interchangeable.

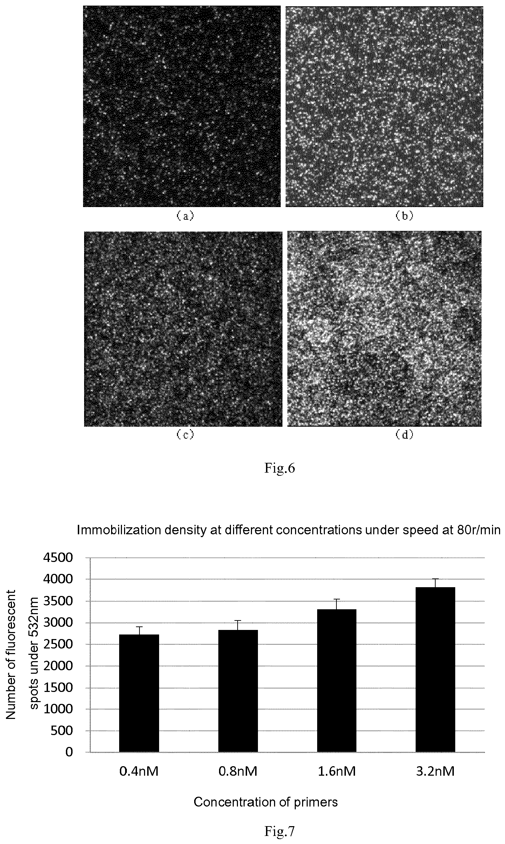

Repeated three times and counted the number of immobilized sequences. The results of the fluorescence microscope are shown in Fig. 5 and Fig. 6. Fig. 5 shows the results of fluorescence microscopy when the rotation speed is at 0 rpm / min during the passivation, including a-d, showing the immobilization effect of 0.4 nM, 0.8 nM, 1.6 nM, 3.2 nM T10C50-Cy3. Fig. 6 shows the results of fluorescence microscopy when the rotation speed is at 80 rpm / min during the passivation, including a-d, showing the immobilization effect of 0.4 nM, 0.8 nM, 1.6 nM, 3.2 nM T10C50-Cy3.

Fig. 7 shows the immobilization density of immobilized DNA at different concentration when the rotation speed is 80 rpm / min.

As can be seen from Fig. 5 to 7:
1. It can be seen, when compared to the 532 fluorescence photo at corresponding concentration,that the rotational speed of the shaker to 40-80 rpm / min (preferably 80 rpm / min) during the passivation can significantly eliminate large fluorescent spots;
2. In the passivation process under certain conditions, the immobilization density will increase with the increase of the concentration of the DNA to be immobilized, therefore we can choose the best immobilization concentration based on the immobilization concentration needed. In the subsequent hybridization experiments, when the immobilization density is higher than 4000, too high immobilized DNA concentration will cause too large DNA molecules steric hindrance for DNA hybridization, which will lead to too low hybrid density, so according to hybrid experimental results, we choose a more suitable immobilization concentration 0.8-1.6 nM.

It is to be noted that in the DNA chip immobilization experiment, the examples of the present disclosure label a fluorescent at the 3 'end of the primer, with the aim of better tracking the primer position and exploring a more suitable immobilization density. In the application, the 3 'end of the primer is not fluorescently labeled, allowing the nucleotide to be extended under the catalysis of the polymerase.

It will be appreciated that one skilled in the art can label optical detection labels, such as fluorescent labels, as desired, at other positions outside the 3 'end of the primer (without affecting the extension reaction).

### DNA single molecule sequencing

In particular, in the case of a specific sample, a single molecule sequencing method provided by an example of the present disclosure comprises:
1. Sequencing reaction: before sequencing, the disease-related test sequence is interrupted into a small fragment and labeling a fluorescence molecule at the end thereof. At the same time, a plurality of targeted primers are randomly immobilized to the substrate. The small fragment DNA template is hybridized with the immobilized primers and accurately positioned, and the optical image is collected by a total internal reflection microscope (TIRF) to accurately position the position of the hybridized small fragment DNA template. And then a mixture of monochromatic or multicolor terminal terminator with a cleavable fluorescent label and polymerase is added, incubate, wash, perform optical imaging, and record the position of the present reaction. Reagents are then added to remove the fluorescent molecules at the extended position, wash, cap, and ready for the next nucleotide extension reaction. Real-time sequencing can be achieved by repeated cycles of extension reaction, imaging detection and excising the fluorescent molecules.
2. Image acquisition: image acquisition is performed using a total internal reflection microscopy system (TIRF), because the single molecule signal is very weak, TIRF system can significantly reduce the background noise, thereby enhancing the signal to noise ratio. This system is a double-color laser light system. Before each acquisition of the image, it is necessary to process the sample encapsulated in the microfluidic system according to the chemical flushing process, and then photograph. To prevent the sample from drifting, the positioning information needs to be photographed before each extension reaction, then photograph after base extension to obtain a sequencing signal.
3. Image processing: in step 1, the present disclosure is imaged according to the desired sequencing requirements, and the images obtained in each base extension reaction may have several tens or thousands of fields of view. For the processing of the images, it is necessary to accurately calculate the coordinate position of each reaction and record it. Then the images obtained during each base extension is subjected to position correction by an image processing software, to correct the position drift caused by the experienced chemical flushing process and sample movement. And then the images are overlapped, and the positions of the sequencing reactions are successively superimposed to obtain the base sequence of each position.
4. Bioinformatics analysis: on the basis of the obtained base sequence, the complete sequence of the DNA fragment is obtained by base determination and comparison. Bioinformatics involved to complete the analysis of the biological significance of the fragment, and to help doctors determine the choice of drugs, and to screen of diseases.

There is an article "Single molecule targeted sequencing for cancer gene mutation detection" published in Nature, which is developed by the team of this company. It tested synthetic DNA samples (containing low frequency mutations) by using the above method, the results verified that the method can detect the low frequency and low abundance mutation sites in the sample without amplifying the original sample.

Specifically, the sequencing apparatus used includes a total internal reflection fluorescence (TIRF) microscope, which is equipped with a 60-fold objective lens (oil) (Nikon Ti-E,Japan), a EMCCD camera with a 512 x 512 resolution (Andor, Belfasst, UK), a 2-color laser power source (532 nm, 100 mW; and 640 nm, 100 mW), a stage mounted on a TIRF microscope (ASI, Eugene, OR, USA), which is used to support and control the flow path pool during the sequencing process (Bioptechs, Bulter, PA, USA), a heating device (Bioptechs, Bulter, PA, USA) maintaining the temperature of the sample cell in the flow path pool and the objective lens at 37 ° C.

Specifically, the FCS2 flow path pool used includes an epoxy-modified slide (Schott, Jena, Germany),with a slide thickness of 0.175 mm and a diameter of 40 mm. A spacer is set between the slide and a aqueduct to form a sample cell (3 mm × 23 mm × 0.25 mm) for the sequencing reaction. A Titan EZ valve with 12 channels (IDEX Health & Science, Oak Harbor, WA, USA) connects the sequencing reaction reagents to the inlet of the sequencing flow path pool, and a syringe pump draws the liquid system from the sample cell by suction (TecanMännedorf, Swiss).

In the optical path module of the sequencing apparatus, the Cy3 fluorescent label at the 3 'end of the target DNA fragment is excited by green laser; the Cy3 fluorescent label is a non-cleavable group; the cleavable group ATTO647N fluorescent label modified on primers is excited by red laser; the EMCCD camera system can independently capture the fluorescent images of Cy3 fluorescent label and ATTO647N fluorescent label. The primers are covalently linked to the surface of the slide, the target DNA fragment is complementary to the primer, and the evanescent wave of TIRF only stimulated the area of 200 nm above the surface of the sample cell. Thus, only the DNA template strand in the evanescent wave excitation region can be detected.

The synthetic wild-type EGFR / KRAS / BRAF gene is sequenced and sequenced for 19-30 cycles to cover all of the preset mutations and deletions. In each of the extension reactions, images of 300 fields of view are collected, each having approximately 2200-2500 sequences (reads), about 0.7-0.8 reads/µm². Sequencing results are analyzed and found that the average sequencing depth is 1954 times.

In the single molecule sequencing method example, a synthetic DNA fragment is analyzed, covering 8 mutations in three genes of EGFR, KRAS and BRAF, including 6 point mutations (G719A in EGFR exon 18, T790M in EGFR exon 20, L858R and L861Q in EGFR exon 21, G12S and G13D in KRAS exon 2, V600E in BRAF exon 15), and 2 deletion mutations (Δ E746-A750 and Δ E747-A753 deletion mutations in EGFR exon 19); wild-type and mutant-type sequences are designed for each mutation site; the length of each target template DNA fragment is designed to be 70nt, and Cy3 fluorescent groups are modified at the 3 'end; these synthetic target DNA fragments can be hybridized to the capture primers immobilized on the flow path pool.

The primers used are complementary to the synthetic DNA template described above. In particular, the primers are designed using BatchPrimer3 software with a GC content between 20-80%, a Tm value greater than 65 ° C, a length of 60 n, a dT10 (10 T) and an amino group at the 5 'end, and complementary to the upstream sequence of the mutation sites of the synthetic DNA template. In particular, the designed primers and target DNA fragments were synthesized by Sangon Biotech (Shanghai, China).

In the example of the single molecule sequencing method, a reversible terminator (dNTP-ATTO647N) is the triphosphate nucleotide modified by a detectable fluorescent label (ATTO647N, linking via -S-S-linker) and an inhibiting group. The detectable fluorescent label and the inhibiting group are both cleavable groups.

In the single molecule sequencing method example, the synthesized primer is immobilized on an epoxy-modified slide (chip), comprising the steps of:
1) The primer is incubated at 95 ° C and the chip to be immobilized is purged with a nitrogen gun;
2) The chip is immersed in a immobilized buffer containing 1 nM K2HPO4 (150 mM, pH = 8.5) for 2 hours at 37 ° C and the concentration of the primer in the immobilized buffer is 0.8 nM;
3) and then the chip is washed with 3x SSC + 0.1% Triton, 3x SSC, 150 mM K2HPO4 pH = 8.5;
4) and then the pH = 9.0, 1 M K2HPO4 solution is used to immerse the chip, shaking for 15 hours at room temperature on a shaker at 80rpm (r / min);
5) and the chip is washed sequentially with PBS, 150 mMHepes + 150 mMNaCl, and finally rinse the chip with double distilled water.

In the single molecule sequencing method example, a synthetic DNA template labeled with Cy3 is directed to a sequencing chip flow cell at 5 nM 3 x SSC, pH = 7.0, incubated at 55 ° C for 2 h to form DNA double strand. The sequencing chip is washed by dye rinse buffer 1, and dye rinse buffer 2.

The sequencing process is automatically controlled, including the use of fluid systems and imaging processes of 9 pre-prepared reagents stored at both temperatures. Seven of the nine reagents are chemical- or bio-reaction reagents and are stored at 4 ° C, including four nucleotides (dNTP-ATTO647N) and DNA polymerase mixtures, resection reagent (TCEP, 50 mM), capping reagent (50 mM iodine acetamide) and imaging buffer (HEPES buffer containing 50 mMTrolox, 15 mM DABCO, 50 mMNal, 20 mM glucose and 5 mM glucose oxidase). The other two are dye buffers, rinse buffer 1 (150 mM HEPES, 1 x SSC and 0.1% SDS, pH = 7.0) and rinse buffer 2 (150 mM HEPES containing 150 mMNaCl, pH = 7.0), stored at room temperature.

At the start of sequencing, a mixture of 0.25 µM reversible terminator (one of G, C, T and A) and 20 nMKlenowfragment (Exo-) polymerase (New England Biolabs) is introduced into the flow cell, incubated at 37 ° C for 4 min, washing with rinse buffer 1 and 2 in turn.

Next, the imaging buffer is added to the flow cell to capture 300 fields of view (FOVs). Typically, four images in each field of view (54.6 µ m m x 54.6 µm) are obtained using an exposure time of 0.1 s. After imaging, rinse buffer is used to wash the flow cell. Adding the resection reagent to the flow cell to react for 5 min, and then adding the capping reagent to react for 5 min, and finally wash the flow cell again with rinse buffer. The first cycle of the sequencing reaction is achieved above. The above sequencing cycles are achieved using different reversible terminators. In this example, the order of addition of the terminator is G, C, T, A.

In the single molecule sequencing method example, the bright spot localization algorithm is used to process the collected images, including the positioning and correcting of the images, and obtaining the sequence information of the nucleic acid to be tested.

In the single molecule sequencing method example, the results showed that: 1) the average sequencing accuracy is 95% when the sequencing depth is 1 time, and the average sequencing accuracy is 100% when the depth is 5 times and more; 2) the results of four cycles of sequencing showed that the base substitution error is lower and the average base substitution error is 0.52% relative to the base deletion error; 3) mutations with mutation frequency as low as 3% could be detected.

It's understandable that the length of reads could be increased by increasing the reaction cycles, though the length of reads in this article is about 10bp.

The four kinds of reversible terminators (A, T, C, G) used in the SMTS sequencing are used with the same fluorescent label. It can be understood that the four reversible terminators (A, T, C, G) can be with different fluorescent labels from each other.

## Claims

1. A single molecule sequencing method comprising:
(i) combining a template nucleic acid having a first optical detection label at the 5' and/or the 3'end of the template nucleic acid with a primer to obtain a first complex, wherein the primer being attached to a surface of a substrate;
(ii) imaging the first optical detection label of the first complex to obtain a first image;
(iii) mixing the first complex, polymerase, and one or more nucleotides with a second optical detection label, and thus adding the one or more of nucleotides with a second optical detection label to the first complex by polymerization reaction, thereby obtaining an extension product, wherein the nucleotides with a second optical detection label comprises a second optical detection label, a cleavable group and a nucleotide that are sequentially connected,
wherein either (a) the nucleotide(s) with a second optical detection label is/are a monochromatic reversible terminator, wherein the monochromatic reversible terminator is any one of A, T, C, and G with the same fluorescent label and only one kind of the nucleotides with a second optical detection label is added or (b) the nucleotides with a second optical detection label are a multicolor reversible terminators, wherein the multicolor reversible terminator are at least two of A, T, C, and G with different fluorescent labels, and two or more kinds of the nucleotides with a second optical detection label are added simultaneously;
(iv) imaging the second optical detection label of the extension product to obtain a second image;
(v) removing the cleavable group from the extension product to obtain a second complex;
(vi) repeating a cycle of reaction comprising steps (ii) to (v) one or more times, wherein the second complex of (v) of the previous cycle of reaction becomes the first complex of (ii) of the next cycle of reaction; and
(vii) performing a first correction based on the first image and the second image of a same cycle of reaction, thereby correcting the positional drift of the second image, and determining the template nucleic acid sequence by successively superimposing the second images after the first correction.

2. A method according to claim 1, wherein the first optical detection label is a non-light breakable label.

3. A method according to claim 1, wherein the primer is a nucleotide sequence having a polyT of 10-30 nt at the 5 'end, at least part of the primer is complementary to at least part of the template nucleic acid; and/ or wherein
the primer is attached to a substrate with surface-modified epoxy through an amino group modified at the 5 'end.

4. A method according to claim 1, wherein the first optical detection label at the end of the template nucleic acid is a fluorescent label.

5. A method according to claim 1, wherein the cleavable group is a photo-cleavable group, a chemically-cleavable group and/or an enzyme-catalyzed cleavable group.

6. A method according to any of claims 1 to 5, wherein the template nucleic acid is DNA and / or RNA.

7. A single molecule sequencing apparatus comprising:
a combining module, for combining a template nucleic acid having a first optical detection label at the 5' and/or the 3'end with a primer to obtain a first complex, wherein the primer being attached to a surface of a substrate;
a first imaging module, for imaging the first complex from the combining module to obtain a first image;
a synthesizing module, for mixing the first complex from the combining module, polymerase, and one or more of nucleotides with a second optical detection label, and thus adding the one or more of nucleotides with the second optical detection label to the first complex by polymerase reaction, obtaining an extension product, wherein the nucleotides with the second optical detection label comprises a second optical detection label, a cleavable group and a nucleotide that are sequentially connected;
a second imaging module, for imaging the extension product from the synthesizing module to obtain a second image;
a cleaving module, for removing the cleavable group from the extension product from the synthesizing module to obtain a second complex;
an iterating module, for replacing the first complex from the combining module with the second complex from the cleaving module, and entering in turn the first imaging module, the synthesizing module, the second imaging module and the cleaving module one or more times to determine the template nucleic acid sequence; and
an image processing module, for determining the template nucleic acid sequence by comparing the difference between the first image and the second image, and performing a first correction based on the first image and the second image of the same cycle of reaction based on the positional drift to obtain a corrected second image.

8. An apparatus according to claim 7, wherein the first imaging module is used for imaging the first complex with different light sources to obtain a plurality of first images; or
the second imaging module is used for imaging the extension product with different light sources to obtain a plurality of second images.

## Patentansprüche

1. Einzelmolekülsequenzierungsverfahren, umfassend:
(i) Kombinieren einer Templatnukleinsäure, die eine erste optische Erfassungsmarkierung an dem 5'- und/oder dem 3'-Ende der Templatnukleinsäure aufweist, mit einem Primer, um einen ersten Komplex zu erhalten, wobei der Primer an eine Oberfläche eines Substrats gebunden ist;
(ii) Abbilden der ersten optischen Erfassungsmarkierung des ersten Komplexes, um ein erstes Bild zu erhalten;
(iii) Mischen des ersten Komplexes, einer Polymerase und eines oder mehrerer Nukleotide mit einer zweiten optischen Erfassungsmarkierung und somit Hinzufügen des einen oder der mehreren der Nukleotide mit einer zweiten optischen Erfassungsmarkierung zum ersten Komplex durch Polymerisierungsreaktion, wodurch ein Erweiterungsprodukt erhalten wird, wobei die Nukleotide mit einer zweiten optischen Erfassungsmarkierung eine zweite optische Erfassungsmarkierung, eine spaltbare Gruppe und ein Nukleotid umfassen, die sequenziell verbunden sind,
wobei entweder (a) das Nukleotid/die Nukleotide mit einer zweiten optischen Erfassungsmarkierung ein monochromatischer reversibler Terminator ist/sind, wobei der monochromatische reversible Terminator ein beliebiger von A, T, C und G mit der gleichen fluoreszierenden Markierung ist und nur eine Art der Nukleotide mit einer zweiten optischen Erfassungsmarkierung hinzugefügt wird oder (b) die Nukleotide mit einer zweiten optischen Erfassungsmarkierung ein mehrfarbiger reversibler Terminator sind, wobei der mehrfarbige reversible Terminator mindestens zwei von A, T, C und G mit unterschiedlichen Fluoreszenzmarkierungen ist und gleichzeitig zwei oder mehrere Arten der Nukleotide mit einer zweiten optischen Erfassungsmarkierung hinzugefügt werden;
(iv) Abbilden der zweiten optischen Erfassungsmarkierung des Erweiterungsproduktes, um ein zweites Bild zu erhalten;
(v) Entfernen der spaltbaren Gruppe von dem Erweiterungsprodukt, um einen zweiten Komplex zu erhalten;
(vi) Wiederholen eines Reaktionszyklus, umfassend die Schritte (ii) bis (v) ein oder mehrere Male, wobei der zweite Komplex von (v) des vorherigen Reaktionszyklus zum ersten Komplex von (ii) des nächsten Reaktionszyklus wird; und
(vii) Durchführen einer ersten Korrektur basierend auf dem ersten Bild und dem zweiten Bild eines gleichen Reaktionszyklus, wodurch die Positionsdrift des zweiten Bildes korrigiert wird, und Bestimmen der Templatnukleinsäuresequenz, indem die zweiten Bilder nach der ersten Korrektur nacheinander überlagert werden.

2. Verfahren nach Anspruch 1, wobei die erste optische Erfassungsmarkierung eine nicht durch Licht brechbare Markierung ist.

3. Verfahren nach Anspruch 1, wobei der Primer eine Nukleotidsequenz ist, die ein PolyT von 10-30 Nukleotiden am 5'-Ende aufweist, wobei mindestens ein Teil des Primers zu mindestens einem Teil der Templatnukleinsäure komplementär ist; und/ oder wobei
der Primer an einem Substrat mit oberflächenmodifiziertem Epoxid durch eine Aminogruppe, die am 5'-Ende modifiziert ist, gebunden ist.

4. Verfahren nach Anspruch 1, wobei die erste optische Erfassungsmarkierung am Ende der Templatnukleinsäure eine Fluoreszenzmarkierung ist.

5. Verfahren nach Anspruch 1, wobei die spaltbare Gruppe eine durch Licht spaltbare Gruppe, eine chemisch spaltbare Gruppe und/oder eine enzymkatalysierte spaltbare Gruppe ist.

6. Verfahren nach einem der Ansprüche 1 bis 5, wobei die Templatnukleinsäure DNA und/oder RNA ist.

7. Einzelmolekülsequenzierungsvorrichtung, umfassend:
ein Kombinierungsmodul, zum Kombinieren einer Templatnukleinsäure, die eine erste optische Erfassungsmarkierung an dem 5'- und/oder dem 3'-Ende aufweist, mit einem Primer, um einen ersten Komplex zu erhalten, wobei der Primer an eine Oberfläche eines Substrats gebunden ist;
ein erstes Bildgebungsmodul, zur Bildgebung des ersten Komplexes aus dem Kombinierungsmodul, um ein erstes Bild zu erhalten;
ein Synthetisierungsmodul, zum Mischen des ersten Komplexes aus dem Kombinierungsmodul, einer Polymerase und eines oder mehrerer von Nukleotiden mit einer zweiten optischen Erfassungsmarkierung und somit Hinzufügen des einen oder der mehreren der Nukleotide mit der zweiten optischen Erfassungsmarkierung zum ersten Komplex durch Polymerisierungsreaktion, wodurch ein Erweiterungsprodukt erhalten wird, wobei die Nukleotide mit der zweiten optischen Erfassungsmarkierung eine zweite optische Erfassungsmarkierung, eine spaltbare Gruppe und ein Nukleotid umfassen, die sequenziell verbunden sind;
ein zweites Bildgebungsmodul zur Bildgebung des Erweiterungsproduktes aus dem Synthetisierungsmodul, um ein zweites Bild zu erhalten;
ein Spaltmodul, zum Entfernen der spaltbaren Gruppe aus dem Erweiterungsprodukt aus dem Synthetisierungsmodul, um einen zweiten Komplex zu erhalten;
ein iterierendes Modul, zum Ersetzen des ersten Komplexes aus dem Kombinierungsmodul durch den zweiten Komplex aus dem Spaltmodul und wiederum Eingeben des ersten Bildgebungsmoduls, des Synthetisierungsmoduls
des zweiten Bildgebungsmoduls und des Spaltmoduls ein oder mehrere Male, um die Templatukleinsäuresequenz zu bestimmen; und
ein Bildverarbeitungsmodul, zum Bestimmen der Templatnukleinsäuresequenz durch Vergleichen des Unterschieds zwischen dem ersten Bild und dem zweiten Bild und Durchführen einer ersten Korrektur basierend auf dem ersten Bild und dem zweiten Bild desselben Reaktionszyklus basierend auf der Positionsdrift, um ein korrigiertes zweites Bild zu erhalten.

8. Vorrichtung nach Anspruch 7, wobei das erste Bildgebungsmodul zur Bildgebung des ersten Komplexes mit unterschiedlichen Lichtquellen verwendet wird, um eine Vielzahl von ersten Bildern zu erhalten; oder
das zweite Bildgebungsmodul zur Bildgebung des Erweiterungsproduktes mit unterschiedlichen Lichtquellen verwendet wird, um eine Vielzahl von zweiten Bildern zu erhalten.

## Revendications

1. Procédé de séquençage de molécules uniques comprenant les opérations suivantes:
(i) combiner un acide nucléique matrice ayant un premier marqueur de détection optique à l'extrémité 5' et/ou 3' de l'acide nucléique matrice avec une amorce pour obtenir un premier complexe, l'amorce étant fixée à une surface d'un substrat;
(ii) former une image de la première étiquette de détection optique du premier complexe pour obtenir une première image;
(iii) mélanger le premier complexe, la polymérase, et un ou plusieurs nucléotides avec un second marqueur de détection optique, et ainsi ajouter le ou les nucléotides avec un second marqueur de détection optique au premier complexe par réaction de polymérisation, obtenant ainsi un produit d'extension, dans lequel les nucléotides avec un second marqueur de détection optique comprennent un second marqueur de détection optique, un groupe clivable et un nucléotide qui sont connectés de manière séquentielle,
dans lequel soit (a) le ou les nucléotides avec un second marqueur de détection optique est/sont un terminateur réversible monochromatique, dans lequel le terminateur réversible monochromatique est l'un quelconque de A, T, C et G avec le même marqueur fluorescent et un seul type de nucléotides avec un second marqueur de détection optique est ajouté, soit (b) les nucléotides avec un second marqueur de détection optique sont un terminateur réversible multicolore, où les terminateurs réversibles multicolores sont au moins deux parmi A, T, C et G avec des marqueurs fluorescents différents, et deux types ou plus de nucléotides avec un second marqueur de détection optique sont ajoutés simultanément;
(iv) former une image de la seconde étiquette de détection optique du produit d'extension pour obtenir une seconde image;
(v) éliminer du groupe clivable le produit d'extension pour obtenir un second complexe;
(vi) répéter un cycle de réaction comprenant les étapes (ii) à (v) une ou plusieurs fois, dans lequel le second complexe de (v) du cycle de réaction précédent devient le premier complexe de (ii) du cycle de réaction suivant; et
(vii) effectuer une première correction fondée sur la première image et la seconde image d'un même cycle de réaction, corrigeant ainsi la dérive de position de la seconde image, et déterminer la séquence d'acide nucléique matrice en superposant successivement les secondes images après la première correction.

2. Procédé selon la revendication 1, dans lequel la première étiquette de détection optique est une étiquette non cassable par la lumière.

3. Procédé selon la revendication 1, dans lequel l'amorce est une séquence nucléotidique présentant un polyT de 10-30 nt à l'extrémité 5', au moins une partie de l'amorce est complémentaire d'au moins une partie de l'acide nucléique matrice; et/ou dans lequel
l'amorce est fixée à un substrat avec un époxy modifié en surface par un groupe amino modifié à l'extrémité 5'.

4. Procédé selon la revendication 1, dans lequel le premier marqueur de détection optique à l'extrémité de l'acide nucléique matrice est un marqueur fluorescent.

5. Procédé selon la revendication 1, dans lequel le groupe clivable est un groupe photoclivable, un groupe chimiquement clivable et/ou un groupe clivable par catalyse enzymatique.

6. Procédé selon l'une quelconque des revendications 1 à 5, dans lequel l'acide nucléique matrice est un ADN et/ou un ARN.

7. Appareil de séquençage de molécules uniques comprenant:
un module de combinaison, permettant de combiner un acide nucléique matrice ayant un premier marqueur de détection optique à l'extrémité 5' et/ou 3' avec une amorce pour obtenir un premier complexe, dans lequel l'amorce est fixée à une surface d'un substrat;
un premier module d'imagerie, permettant d'imager le premier complexe provenant du module de combinaison afin d'obtenir une première image;
un module de synthèse, permettant de mélanger le premier complexe provenant du module de combinaison, la polymérase, et un ou plusieurs nucléotides avec un second marqueur de détection optique, et ainsi ajouter le ou les nucléotides avec le second marqueur de détection optique au premier complexe par réaction de polymérase, obtenant un produit d'extension, dans lequel les nucléotides avec le second marqueur de détection optique comprennent un second marqueur de détection optique, un groupe clivable et un nucléotide qui sont séquentiellement connectés;
un second module d'imagerie, permettant d'imager le produit d'extension provenant du module de synthèse afin d'obtenir une seconde image;
un module de clivage, permettant d'enlever le groupe clivable du produit d'extension provenant du module de synthèse pour obtenir un second complexe;
un module d'itération, permettant de remplacer le premier complexe du module de combinaison par le second complexe du module de clivage, et entrer à son tour dans le premier module d'imagerie, le module de synthèse et le module de clivage.
le second module d'imagerie et le module de clivage permettant une ou plusieurs fois de déterminer la séquence d'acide nucléique matrice; et
un module de traitement d'image, permettant de déterminer la séquence d'acide nucléique matrice en comparant la différence entre la première image et la seconde image, et en effectuant une première correction fondée sur la première image et la seconde image du même cycle de réaction sur la base de la dérive de position pour obtenir une seconde image corrigée.

8. Appareil selon la revendication 7, dans lequel le premier module d'imagerie permet d'imager le premier complexe avec différentes sources de lumière pour obtenir une pluralité de premières images; ou
le second module d'imagerie permet d'imager le produit d'extension avec différentes sources de lumière pour obtenir une pluralité de secondes images.
